(19) Europäisches Patentamt
European Patent Office
Office européen des brevets

(11) Veröffentlichungsnummer : **0 200 674**
**B1**

(12) # EUROPÄISCHE PATENTSCHRIFT

(45) Veröffentlichungstag der Patentschrift : **25.05.88**

(51) Int. Cl.[4] : **C 07 C153/07**, C 07 C153/09, C 08 L 63/00

(21) Anmeldenummer : **86810047.0**

(22) Anmeldetag : **27.01.86**

(54) **(Acylthiopropyl)-Polyphenole.**

(30) Priorität : **01.02.85 CH 452/85**

(43) Veröffentlichungstag der Anmeldung : **05.11.86 Patentblatt 86/45**

(45) Bekanntmachung des Hinweises auf die Patenterteilung : **25.05.88 Patentblatt 88/21**

(84) Benannte Vertragsstaaten : **AT CH DE FR GB IT LI NL**

(56) Entgegenhaltungen : **Keine Entgegenhaltungen**

(73) Patentinhaber : **CIBA-GEIGY AG**
**Klybeckstrasse 141**
**CH-4002 Basel (CH)**

(72) Erfinder : **Lehmann, Hans**
**Steinackerstrasse 11**
**CH-4147 Aesch (CH)**
Erfinder : **Zahir, Sheik Abdul-Cader, Dr.**
**Elsternstrasse 12**
**CH-4104 Oberwil (CH)**

**EP 0 200 674 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft (Acylthiopropyl)-Polyphenole, die Herstellung dieser Polyphenole, Zusammensetzungen enthaltend ein härtbares Epoxidharz und diese Verbindungen, deren Verwendung als Einkomponentenklebstofformulierungen, sowie die aus diesen Zusammensetzungen erhaltenen gehärteten Produkte.

Epoxidharze sind wohlbekannt und werden mit den unterschiedlichsten Härtern zur Bildung gehärteter Produkte umgesetzt.

Für viele Anwendungsfälle ist es erwünscht, eine Epoxidzusammensetzung verfügbar zu haben, die leicht härtbar aber genügend lagerfähig ist, um vor Gebrauch vermischt werden zu können. Deshalb ist es unerlässlich, dass die Komponenten, d. h. das Epoxidharz und der Härter, für angemessene Zeiträume nach dem Vermischen nebeneinander bestehen können.

Viele bekannte Härter, die bei erhöhten Temperaturen wirksame und rasche Härtung ergeben würden, sind dabei unzureichend, da sie diese Forderung nicht erfüllen und dazu neigen, bei der Einarbeitung in das Epoxidharz eine Gelierung einzuleiten.

Zur Lösung dieses Problems wurden erhebliche Anstrengungen zur Entwicklung latenter Härter unternommen, d. h. Härter, die bei etwa Raumtemperatur mit den Harzen nicht reaktionsfähig sind, aber bei erhöhten Temperaturen mit ihnen rasch reagieren.

Die Verfügbarkeit solcher latenter Härter ermöglicht es, Epoxidharzzusammensetzungen herzustellen, die lange lagerbeständig sind und dabei zu rascher Härtung beim Erhitzen befähigt sind.

Merkaptane und Phenole sind bekannte Härter für Epoxidharze. Bislang wurde aber nicht versucht, beide funktionellen Gruppen in einem Molekül zu vereinigen und darüber hinaus auch noch den Thiolrest mit einer Schutzgruppe zu versehen. Eine geeignet gewählte Schutzgruppe spaltet unter den Reaktionsbedingungen der Härtung ab, so dass beide reaktiven Gruppen für den Härtungsvorgang zur Verfügung stehen.

o-Acylthiopropylphenole sind aus der US-PS 3,443,012 als Pestizide bekannt. Eine Verwendung als Härter für Epoxidharze ist dort nicht erwähnt. Ausserdem handelt es sich bei den vorbeschriebenen Verbindungen um einkernige Monophenole.

Die vorliegende Erfindung betrifft Verbindungen der Formel I und II

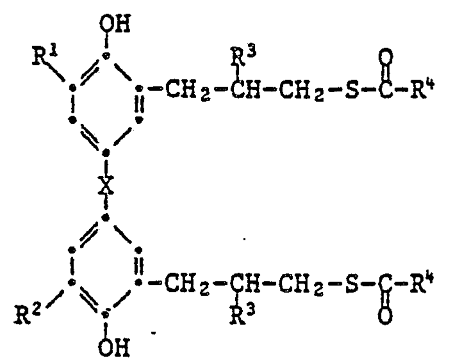

(I)

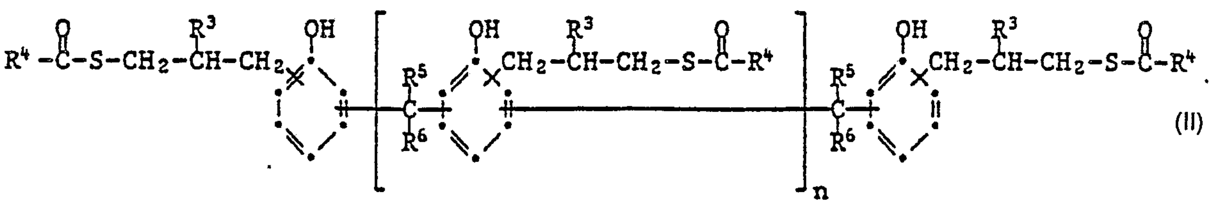

(II)

worin $R^1$ und $R^2$ unabhängig voneinander Wasserstoff, $C_1$-$C_{18}$Alkyl, Cyclohexyl, Phenyl, Benzyl oder Tolyl bedeuten oder ein Rest $—CH_2—CHR^3—CH_2—S—CO—R^4$ sind, $R^3$ Wasserstoff oder Methyl ist, $R^4$ $C_1$-$C_{18}$Alkyl, Cyclohexyl, Phenyl, Benzyl oder Tolyl ist, X $—CR^5R^6—$, $—S—$, $—SO—$, $—SO_2—$ oder $—(CH_3)C[—(CH_2)_m—COOR^7—]$ bedeutet, $R^5$ und $R^6$ unabhängig voneinander Wasserstoff oder $C_1$-$C_6$Alkyl sind, $R^7$ $C_1$-$C_{18}$-Alkyl ist, m 1 oder 2 bedeutet und n eine ganze Zahl von 1 bis 10 ist.

$R^1$, $R^2$, $R^4$ und $R^7$ sind als $C_1$-$C_{18}$Alkyl geradkettig oder verzweigt, bevorzugt geradkettig. Bei diesen Resten handelt es sich beispielsweise um Methyl, Ethyl, Propyl, Isopropyl, n-Butyl, sek.Butyl, tert.Butyl, n-Pentyl, n-Hexyl, n-Heptyl, n-Octyl, n-Nonyl, n-Decyl, n-Dodecyl, n-Tetradecyl, n-Hexadecyl oder n-Octadecyl, sowie um 1,1,3,3-Tetramethylbutyl oder 2-Ethylhexyl.

Bevorzugt werden kurze, geradkettige Alkylreste mit 1 bis 6 Kohlenstoffatomen und ganz besonders Methyl.

Als $C_1$-$C_6$Alkyl bedeuten $R^5$ und $R^6$ beispielsweise Methyl, Ethyl, n-Propyl, n-Butyl, n-Pentyl oder n-Hexyl. Bevorzugt wird Methyl.

Bevorzugt werden Verbindungen der Formel I oder II, worin $R^3$ Wasserstoff bedeutet.

Ganz besonderes Interesse finden Verbindungen der Formel I, worin $R^1$ und $R^2$ unabhängig voneinander Wasserstoff oder $C_1$-$C_{12}$Alkyl sind, $R^3$ Wasserstoff bedeutet, $R^4$ $C_1$-$C_4$Alkyl ist, X eine Gruppe $—CR^5R^6—$ darstellt und worin $R^5$ und $R^6$ unabhängig voneinander Wasserstoff oder Methyl sind.

Ebenfalls von Interesse sind Verbindungen der Formel I, worin $R^1$, $R^2$ und $R^3$ Wasserstoff bedeuten, $R^4$ Methyl ist und X eine Gruppe $—CH_2—$, $—CH(CH_3)—$ oder $—C(CH_3)_2—$ darstellt.

Ganz besonders bevorzugt werden Verbindungen der Formel I, worin $R^1$ und $R^2$ eine Gruppe $—CH_2—CHR^3—CH_2—S—CO—R^4$ darstellen.

Beachtung finden ferner Verbindungen der Formel II, worin $R^3$ Wasserstoff ist, $R^4$ Methyl bedeutet, $—CR^5R^6—$ eine gruppe $—CH_2—$, $—CH(CH_3)—$ oder $—C(CH_3)_2—$ darstellt und worin n eine ganze Zahl von 1 bis 4 ist.

Die Gruppen $—CH_2—CHR^3—CH_2—S—CO—R^4$ bzw. $—CR^5R^6—$ im Novolak der Formel II befinden sich bevorzugt in ortho- oder para-Position zur phenolischen Hydroxygruppe ; ganz besonders bevorzugt befinden sich die Gruppen $—CH_2—CHR^3—CH_2—S—CO—R^4$ in ortho-Position.

$R^1$ und $R^2$ sind vorzugsweise Wasserstoff oder auch Methyl, ganz besonders jedoch Wasserstoff.

$R^3$ ist bevorzugt Wasserstoff, $R^4$ bedeutet vorzugsweise Methyl.

X ist bevorzugt $—CH_2—$ oder $—C(CH_3)_2—$, ganz besonders jedoch $—C(CH_3)_2—$.

Weiterhin bevorzugt als Gruppe X werden $—S—$ oder $—SO_2—$ oder $—(CH_3)C[—CH_2—COOCH_3]—$ oder $—(CH_3)C[—CH_2—CH_2—COOCH_3]—$.

Die Verbindungen der Formel I oder II können erfindungsgemäss beispielsweise dadurch hergestellt werden, indem man Verbindungen der Formel III oder IV

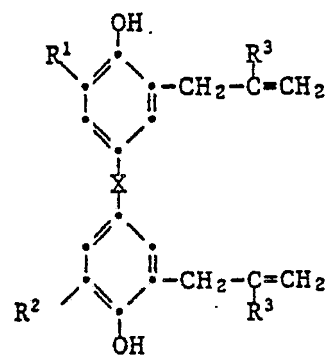

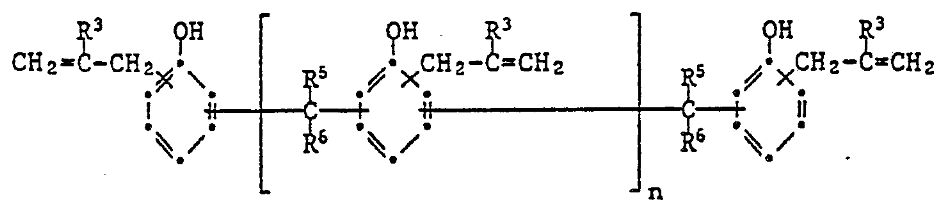

mit einem dem Gehalt an allylständigen Doppelbindungen im wesentlichen entsprechenden molaren Anteil an Thiocarbonsäuren der Formel V

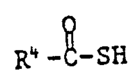

in Gegenwart eines Radikalbildners umsetzt ; dabei besitzen die Reste $R^1$, $R^2$, $R^3$, $R^4$, $R^5$, $R^6$ und X, sowie der Index n die weiter oben definierte Bedeutung, wobei $R^1$ und $R^2$ allerdings anstelle der Bedeutung $—CH_2—CHR^3—CH_2—S—CO—R^4$ in diesem Falle $—CH_2—CR^3 = CH_2$ sind.

Die Bis- oder Polyallylphenole bzw. -methallylphenole der Formeln III oder IV sind bekannte Verbindungen oder können nach an sich bekannten Verfahren hergestellt werden.

Die Thiocarbonsäuren der Formel V sind ebenfalls bekannte Verbindungen und können ebenso nach an sich bekannten Verfahren erhalten werden. Vorzugsweise setzt man ein entsprechendes Carbonsäureanhydrid, bevorzugt Essigsäureanhydrid, mit Schwefelwasserstoff oder einem wasserlöslichen Metallsulfid in alkalischer wässriger Lösung um, isoliert das entstandene Gemisch aus Carbonsäure und Thiocarbonsäure und verwendet dieses ohne weitere Auftrennung in der nachfolgenden Umsetzung mit dem (Meth)allylphenol.

Die Menge der einzusetzenden Thiocarbonsäure V richtet sich nach der Anzahl der Allyl- oder Methallylgruppen im Ausgangsmaterial III oder IV. In der Regel setzt man äquimolare Mengen Thiocarbonsäure ein, bezogen auf die Allylgruppen. Es kann aber durchaus ein Ueberschuss oder auch ein Unterschuss an Thiocarbonsäure verwendet werden.

Verwendet man einen Unterschuss an Thiocarbonsäure, so erfolgt nur eine teilweise Umsetzung der Allylgruppen der Verbindungen III oder IV. Solche teilweise thioacylierten Produkte, insbesondere teilweise umgesetzte Novolake IV, fallen ebenfalls unter den Rahmen dieser Erfindung.

Solche teilweise thioacylierten Novolake IV sind Gemische von Verbindungen unterschiedlicher Kettenlänge und unterschiedlichen Thioacetylierungsgrads. Im Mittel sollten mindestens 50 % der Allylgruppen umgesetzt sein.

Die Umsetzung zum Endprodukt I oder II wird radikalisch initiiert. Dazu bestrahlt man beispielsweise das Reaktionsgemisch gegebenenfalls in Anwesenheit eines Katalysators mit kurzwelligem Licht oder man erwärmt das Gemisch, vorzugsweise in Gegenwart eines Radikalbildners. Die Reaktion lässt sich aber auch rein thermisch durchführen, vorzugsweise in Anwesenheit eines Radikalbildners.

Als Radikalbildner eigenen sich beispielsweise organische Peroxide, wie Benzoylperoxid, Acetylperoxid oder Cumylhydroperoxid, und insbesondere Azoverbindungen. Von diesen wiederum sind insbesondere solche bevorzugt, bei denen die Azogruppe beidseitig an tertiäre C-Atome gebunden ist, die neben Alkylgruppen noch Nitril- oder Estergruppen tragen. So ist beispielsweise α,α-Azoisobuttersäuredinitril (AIBN) ein wichtiger Vertreter dieser Verbindungsklasse.

Bei der photoinitiierten Reaktion sind als gegebenenfalls einsetzbare Katalysatoren z. B. Benzoinether, Benzilketale, ω-Dialkoxy-acetophenon-Derivate oder aromatische Keton-Amin-Kombinationen geeignet.

Die Menge an gegebenenfalls einsetzbarem Radikalbildner ist nicht kritisch und kann in weiten Grenzen schwanken ; sie beträgt vorzugsweise weniger als 10 Mol% des Anteils der Allyl- oder Methallylgruppen im Reaktionsgemisch.

Die Umsetzung der Verbindung III und V oder IV und V kann in An- oder Abwesenheit eines Lösungsmittels erfolgen.

Die gegebenenfalls verwendeten Lösungsmittel müssen gegenüber den Reaktionspartnern inert sein und diese lösen können. So eignen sich beispielsweise aliphatische oder aromatische Kohlenwasserstoffe, wie Hexan, Benzol, Toluol oder Xylol, oder chlorierte Kohlenwasserstoffe, wie Dichlormethan oder Chlorbenzol, sowie Ether, wie Dioxan oder Diethylether, oder aprotische Lösungsmittel, wie Dimethylformamid, für diese Aufgabe. Die Reaktionstemperatur bewegt sich im allgemeinen je nach Reaktionsführung und Reaktionspartner im Temperaturbereich von — 10 °C bis 250 °C.

Vorzugsweise führt man die Umsetzungen des Allylphenols mit der Thiocarbonsäure rein thermisch, bei Temperaturen zwischen 40 und 80 °C unter Schutzgas, beispielsweise $N_2$, in Abwesenheit eines Lösungsmittels und in Anwesenheit eines Radikalbildners aus. Als Radikalbildner bei dieser Variante setzt man insbesondere AIBN ein, obgleich auch andere Radikalbildner sich für diesen Zweck eignen. Bei Verwendung von AIBN ist die Ausbeute an Acylthiopropylverbindung besonders hoch.

Die erfindungsgemässen Phenole können aus dem Reaktionsgemisch in der üblichen Weise isoliert werden. Sie lassen sich beispielsweise durch Destillation, fraktionierte Kristallisation oder Extraktion entfernen, wobei die Extraktion vorzugsweise mit einer wässrigen alkalischen Lösung durchgeführt wird.

Die Verbindung der Formel I und II lassen sich als latente Härter für Epoxidharze verwenden.

Die Erfindung betrifft daher auch Zusammensetzungen enthaltend

a) ein Epoxidharz mit durchschnittlich mehr als einer Epoxidgruppe im Molekül oder ein noch schmelzbares und/oder lösliches härtbares Vorkondensat des Epoxidharzes (sogenannte « B-Stufe »), und

b) mindestens eine Verbindung der Formel I und/oder II, und

c) gegebenenfalls weitere übliche Zusätze, wie z. B. Katalysatoren (Härtungsbeschleuniger) ; sowie die daraus durch Erwärmen erhältlichen gehärteten Produkte.

Bevorzugt werden Zusammensetzungen enthaltend a) das Epoxidharz, und b) mindestens eine Verbindung der Formel I.

Die zu verwendeten Epoxidharze haben vorzugsweise im Durchschnitt mehr als eine Epoxidgruppe pro Molekül. Insbesondere seien genannt :

Alicyclische Polyepoxide, wie Epoxyethyl-3,4-epoxycyclohexan (Vinyl-cyclohexendiepoxid), Limonendiepoxid, Dicyclopentadiendiepoxid, Bis(3,4-epoxycyclohexylmethyl)adipat, 3′,4′-Epoxycyclohexylmethyl-3,4-epoxycyclohexancarboxylat, 3′,4′-Epoxy-6′-methylcyclohexylmethyl-3,4-epoxy-6-methylcyclohexancarboxylat, 3-(3′,4′-Epoxycyclohexyl)-2,4-dioxaspiro[5,5]-8,9-epoxyundecan, 3-Glycidyloxyethoxyethyl-2,4-dioxaspiro[5,5]-8,9-epoxyundecan.

Di- oder Polyglycidylether von mehrwertigen Alkoholen, wie 1,4-Butandiol oder Polyalkylenglykolen, wie Polypropylenglykole, Di- oder Polyglycidylether von cycloaliphatischen Polyolen, wie 2,2-Bis(4′-hydroxycyclohexyl)propan, Di- oder Polyglycidylether von mehrwertigen Phenolen, wie Resorcin, Bis(4-hydroxyphenyl)methan (Bisphenol F), 2,2-Bis(4′-hydroxyphenyl)propan (Bisphenol A), 2,2-Bis(4′-hydroxy-3′,5′-dibromphenyl)propan, 1,1,2,2-Tetrakis(4′-hydroxyphenyl)ethan, oder unter sauren Bedingungen erhaltene Kondensationsprodukte von Phenolen mit Formaldehyd, wie Phenol-Novolake und Kresol-Novolake ; ferner Di- oder Poly(β-methylglycidyl)ether der oben angeführten Polyalkohole und Polyphenole.

Polyglycidylester und Poly(β-methylglycidyl)ester von mehrwertigen Carbonsäuren, wie Phthalsäure, Terephthalsäure, Tetrahydrophthalsäure und Hexahydrophthalsäure.

N-Glycidylderivate von Aminen, Amiden und heterocyclischen Stickstoffbasen, wie N,N-Diglycidylanilin, N,N-Diglycidyltoluidin, N,N,N',N'-Tetraglycidyl-bis(4-aminophenyl)methan, Triglycidylisocyanurat, N,N'-Diglycidylethylharnstoff, N,N'-Diglycidyl-5,5-dimethylhydantoin, N,N'-Diglycidyl-5-isopropylhydantoin, N,N'-Diglycidyl-5,5-dimethyl-6-isopropyl-5,6-dihydrouracil.

Besonders bevorzugt sind Polyglycidylether von Phenol- oder Kresol-Formaldehyd Novolaken sowie Diglycidylether von Bisphenol A und Bisphenol F.

Geeignete Katalysatoren (Beschleuniger) sind z. B. tertiäre Amine, deren Salze oder quaternäre Ammoniumverbindungen, z. B. Benzyldimethylamin, 2,4,6-Tris(dimethylaminomethyl)phenol, 1-Methylimidazol, 2-Ethyl-4-methylimidazol, 2-Phenylimidazol, 4-Aminopyridin, Tripentylammoniumphenolat ; oder Alkalimetallalkoholate, wie z. B. Natriumhexantriolat. Die Umsetzung (Härtung) der erfindungsgemässen Mischungen wird zweckmässig im Temperaturintervall von 50 °C bis 300 °C, bevorzugt von 150-300 °C, durchgeführt.

Der bevorzugte Härtungsbeschleuniger ist 2-Phenylimidazol.

Man kann die Härtung in bekannter Weise auch zwei- oder mehrstufig durchführen, wobei die erste Härtungsstufe bei niedriger Temperatur und die Nachhärtung bei höherer Temperatur durchgeführt wird.

In der Regel wird eine zweistufige Härtung in der Weise durchgeführt, dass die Härtungsreaktion zunächst vorzeitig abgebrochen bzw. die erste Stufe bei wenig erhöhter Temperatur durchgeführt wird, wobei ein noch schmelzbares und/oder lösliches, härtbares Vorkondensat (sogenannte « B-Stufe ») aus der Epoxid-Komponente (a) und der Härterkomponente (b) erhalten wird. Ein derartiges Vorkondensat kann z. B. zur Herstellung von « Prepregs », Pressmassen oder Sinterpulvern dienen.

Die erfindungsgemässen Zusammensetzungen enthaltend a) ein Epoxidharz mit durchschnittlich mehr als einer Epoxidgruppe im Molekül, b) mindestens eine Verbindung der Formel I oder II und c) gegebenenfalls einen Härtungsbeschleuniger bilden überraschenderweise lösliche B-Stufen, wenn diese Harz/Härter Mischungen bei Raumtemperatur gelagert werden. Solche Mischungen sind über längere Zeit (Monate) lagerfähig und können somit als Einkomponenten-Klebstofformulierungen verwendet werden.

Der Ausdruck « Härten », wie er hier gebraucht wird bedeutet die Umwandlung der löslichen, entweder flüssigen der schmelzbaren Epoxidharze in feste, unlösliche und unschmelzbare, dreidimensional vernetzte Produkte bzw. Werkstoffe, und zwar in der Regel unter gleichzeitiger Formgebung zu Formkörpern, wie Giesskörpern, Presskörpern und Schichtstoffen, zu Imprägnierungen, Beschichtungen, Lackfilmen oder Verklebungen.

Die erfindungsgemässen Gemische können durch einfaches Zusammenrühren der Komponenten und vorsichtiges Aufwärmen bis zum Lösen der Komponenten hergestellt werden. Falls ein festes Epoxidharz vorliegt, wird dieses vorübergehend zum Schmelzen erwärmt, worauf man darin den Härter und gegebenenfalls den Härtungsbeschleuniger und/oder weitere Zusätze löst.

Die erfindungsgemässen härtbaren Mischungen können ferner vor der Härtung in irgendeiner Phase mit üblichen Modifizierungsmitteln, wie Streck-, Füll- und Verstärkungsmitteln, Pigmenten, Farbstoffen, Weichmachern, Verlaufmitteln, Thioxotropiermitteln, Flexibilisatoren, flammhemmenden Stoffen oder Formtrennmitteln versetzt werden.

Als Streckmittel, Verstärkungsmittel, Füllmittel und Pigmente, die in den erfindungsgemässen härtbaren Mischungen eingesetzt werden können, seien z. B. genannt : Steinkohlenenteer, Bitumen, flüssige Cumaron-Inden-Harze, Textilfasern, Glasfasern, Asbestfasern, Borfasern, Kohlenstoffasern, Cellulose, Polyester, Polyamide, Polyethylenpulver, Polypropylenpulver, Holzmehl, Quarzmehl, mineralische Silikate, wie Glimmer, Asbestmehl, Schiefermehl, Kaolin, Kieselsäureaerogel, Lithopone, Schwerspat, Titandioxid, Russ, Graphit, Oxidfarben, wie Eisenoxid, oder Metallpulver, wie Aluminiumpulver oder Eisenpulver.

Als Weichmacher können für die Modifizierung der härtbaren Mischungen z. B. Dibutyl-, Dioctyl- oder Dinonylphthalat, Trikresylphosphat, Trixylenylphosphat und Diphenoxyethylformal eingesetzt werden.

Als Verlaufmittel beim Einsatz der härtbaren Mischungen speziell im Oberflächenschutz, kann man z. B. Silikone, flüssige Acrylharze, Celluloseacetobutyrat, Polyvinylbutyral, Wachse, Stearate etc. (welche z. T. auch als Formtrennmittel Anwendung finden) zusetzen.

Als Flexibilisatoren eignen sich z. B. Oligoestersegmente, Polyester, Thermoplaste und Butadien-Acrylnitril-Oligomere, wie Hycar® von der Firma Goodrich.

Die erfindungsgemässen härtbaren Gemische zeichnen sich durch gute Lagerstabilität, lange Verarbeitungszeiten und durch gute Durchhärtung auch bei offener Härtung in dünner Schicht aus. Die gehärteten Produkte besitzen hohe Wärmeformbeständigkeit, gute Heisswasser- und Chemikalien-Beständigkeit und gute Wärmebeständigkeit. Sie zeichnen sich jedoch besonders durch gute Heisswasserbeständigkeit aus, was die härtbaren Gemische vor allem für Klebstofformulierungen interessant erscheinen lässt. Als besonders überraschend ist die Langzeitbeständigkeit von Verklebungen bei der Heisswasserlagerung anzusehen.

Die erfindungsgemässen härtbaren Gemische finden ihren Einsatz vor allem auf den Gebieten des Oberflächenschutzes, der Elektrotechnik, der Laminierverfahren, der Klebstofftechnik und im Bauwesen. Sie können in jeweils dem speziellen Anwendungszweck angepasster Formulierung, im ungefüllten oder gefüllten Zustand, gegebenenfalls in Form von Lösungen der Emulsionen, als Anstrichmittel, lösungsmittelfreie Beschichtungen, Sinterpulver, Pressmassen, Spritzgussformulierungen, Tauchharze, Giessharze, Schaumstoffe, Klebstoffe, Filme, Folien, Bindemittel, Werkzeugharze, Laminierharze, Dichtungs- und

Spachtelmassen, Bodenbelegsmassen und Bindemittel für mineralische Aggregate verwendet werden.
Insbesondere betrifft die Erfindung die Verwendung von Mischungen enthaltend a) ein Epoxidharz mit durchschnittlich mehr als einer Epoxidgruppe im Molekül, und b) mindestens eine Verbindung der Formel I und/oder II als Einkomponenten-Klebstofformulierung.

## Herstellungsbeispiele

1a) o,o'-Bis-(3-acetylthiopropyl)-bisphenol A

666,8 g 2,2'-Bis-(3-allyl-4-hydroxyphenyl)-propan werden in einem Reaktionskolben, ausgestattet mit Rührer, Tropftrichter und Stickstoffeinlassrohr vorgelegt und unter Stickstoffstrom auf 75 °C erwärmt. Es werden 4,92 g AIBN zugefügt und anschliessend 780 g Thioessigsäure im Zeitraum einer Stunde zugetropft. Die Temperatur im Reaktionskolben wird bei 75 °C gehalten und alle 20 Minuten werden weitere 4,92 g AIBN zugegeben, bis die Gesamtmenge 19,68 g erreicht. Nach dem Zutropfen der Thioessigsäure wird weitere 4 Stunden bei 75 °C gerührt. Dann wird nochmals eine Portion von 4,92 g AIBN zugegeben und für weitere 3 Stunden gerührt.
Anschliessend wird die Reaktionsmischung im Rotationsverdampfer eingeengt. Man erhält 1 036 g einer gelben Paste.

a) Elementaranalyse :

berechnet : C 65,19 H 7,00 S 13,92 %
gefunden : C 64,5 H 6,96 S 13,2 %

b) 100 MHz $^1$H-NMR Spektrum.

Man findet keine olefinischen Protonensignale in der Region zwischen 5 und 6 ppm (Standard : TMS). Es sind also keine Allylgruppen mehr nachweisbar.

Signale bei :
2,3 ppm (2 Protonen ; $-S-CO-CH_3$)
1,8 ppm (2 Protonen ; $-S-C-CH_2-C$-Phenyl)
2,6 ppm (2 Protonen ; $-S-CH_2-C-C$-Phenyl)
2,8 ppm (2 Protonen ; $-S-C-C-CH_2$-Phenyl).

1b) Reinigung des Rohprodukts von Beispiel 1a)

360,8 g des pastenförmigen Produktes von Beispiel 1a) werden in 100 ml heissem Xylol gelöst. Es wird 1 g Aktivhohle zugegeben, die Lösung filtriert und anschliessend bei 5 °C stehen gelassen. Die ausgefallenen Kristalle werden abfiltriert und bei 100 °C in Vakuum getrocknet (50 mbar). Man erhält 142 g weisse Kristalle vom F.P. 118,1-119,5 °C.

Analyse des gereinigten Produktes :
a) Elementaranalyse :

berechnet : C 65,19 H 7,00 S 13,92 O 13,89 %
gefunden : C 65,88 H 7,07 S 13,43 O 13,79 %

b) 250 MHz $^1$H-HMR Spektrum

Signale bei (Standard : TMS) :

| Chem. Verschiebung (ppm) | 0,6 | 0,8 | 1,3 | 1,6 |
|---|---|---|---|---|
| Zahl der Protonen (gemessen) | 6 | 4.5 | 6 | 4,1 |
| Zahl der Protonen (erwartet) | 6 | 4 | 6 | 4 |
| signalerzeugende Gruppen | $-C(CH_3)_2$ | $-C-CH_2-C-$ | $-S-CO-CH_3$ | $-C-CH_2-S-CO-$ |

Fortsetzung

| | | | |
|---|---|---|---|
| Chem. Verschiebung (ppm) | 2,8 | 4,85 | 6,6-7,0 |
| Zahl der Protonen (gemessen) | 4,2 | 2 | 6,3 |
| Zahl der Protonen (erwartet) | 4 | 2 | 6 |
| signalerzeugende Gruppen | Phenyl-$CH_2$-C-C- | -OH | Phenyl |

2a) Herstellung eines (o-Acetylthiopropyl)-phenol Formaldehyd Novolaks

Nach dem Verfahren gemäss Beispiel 1 werden 419,9 g eines 2-Allylphenol Formaldehyd Novolaks (Allylgehalt : 2,36 Val) mit 179,9 Thioessigsäure (2,36 Mol) und 10,2 g AIBN unter Stickstoff bei 80 °C umgesetzt. Das AIBN wird gemäss Beispiel 1 in fünf Portionen zu jeweils 2,04 g zugegeben.

Man erhält 589,2 g eines Produktes (Ausbeute : 98,2 % der Theorie).

Die $^1H$ NMR Signale (250 MHz) der Allylprotonen des Ausgangsproduktes im Bereich von 5,1-5,2 ppm bzw. 5,9-6,1 ppm (gegen TMS) sind im $^1H$ NMR Spektrum des Endproduktes verschwunden. Dafür tritt ein Signal der Protonen der Acetylgruppe auf (bei 2,3 ppm).

2b) Reinigung des Rohproduktes

177,2 g des Rohproduktes von Beispiel 2a) werden mit 200 ml Dichlormethan und 220 ml wässrigem Ethanol (Wassergehalt : 30 Vol.%) versetzt und die überstehende Phase abdekantiert. Es werden nochmals 220 ml wässriges Ethanol und 100 ml Wasser zugegeben und abdekantiert.

Der zurückbleibende Novolak wird noch zweimal mit Wasser gewaschen und über $Na_2SO_4$ getrocknet. Anschliessend trocknet man das Produkt noch bei 40 °C (17 mm Hg) bzw. 60 °C (3 mm Hg) am Rotationsdampfer. Man erhält 164 g eines gereinigten Endproduktes.

## Anwendungsbeispiele

A. Messung der Adhäsion von Klebstofformulierungen auf Al-Oberflächen

Es werden Klebstofformulierungen bestehend aus dem jeweiligen Epoxidharz, Härter und 2-Phenylimidazol als Härtungsbeschleuniger hergestellt.

Dazu wird das Flüssigepoxidharz bei 85 °C mit dem Härter vermischt. Nach dem Auflösen wird auf 50 °C abgekühlt und der Härtungsbeschleuniger zugegeben. Im Falle des Festepoxidharzes wird der Härter bei 100 °C gelöst und der Härtungsbeschleuniger bei 70 °C zugegeben. Die Mischung wird anschliessend auf Al-Folie gegossen und pulverisiert.

Mit diesen Formulierungen werden Klebverbindungen zwischen Aluminiumflächen hergestellt. Dazu werden Ausbohrungen in einer Aluminiumplatte, die einen definierten Durchmesser und eine definierte Tiefe besitzen, mit der Harzmischung gefüllt. Anschliessend werden Aluminiumzylinder definierten Durchmessers auf dieser Unterlage fixiert. Die Klebstellen werden für zwei Stunden bei 120 °C gehärtet.

Die Messung der Adhäsion der Klebstelle erfolgt mittels eines Twistometers (vgl. Adhesion 3, edited by K.W. Allen ; Applied Science Publishers Ltd. ; Barking (Essex) ; 1978).

Dazu wird die Al-Grundplatte fixiert und auf den Al-Zylinder wird mittels eines Hebelarms eine definierte Torsionskraft ausgeübt. Aus der maximalen Torsionskraft, die zum Bruch der Klebstelle führt, lässt sich die Adhäsion ermitteln.

In Tabelle 1 sind die Adhäsionswerte der untersuchten Flüssig- und Festepoxidkleber angegeben, in denen 2,2'-Bis-(3-acetylthiopropyl-4-hydroxyphenyl)-propan (Beispiel 1) als Härter enthalten ist ; daneben enthält diese Tabelle die Glasumwandlungstemperaturen $T_g$ der ausgehärteten Klebmassen (differentialthermoanalytisch bestimmt).

In Tabelle 2 findet man die Twistometerdaten von Epoxidklebern enthaltend unterschiedliche Mengen 2-Acetylthiopropylphenol-Formaldehyd Novolak von Beispiel 2 in Kombination mit 2-Phenylimidazol als Härtungsbeschleuniger. Ferner enthält die Tabelle die Viskosität der Mischung bei 40 °C.

(Siehe Tabelle 1 Seite 8 f.)

Tabelle 1

| Typ Epoxidharz | Formulierung (Gew.Teile) | | | Molverhältnis Härter/Epoxidäquivalent Δ | Adhäsion (N/mm²) | $T_g$ *) (°C) |
|---|---|---|---|---|---|---|
| | Epoxidharz | Härter | Härtungsbeschleuniger | | | |
| Flüssigepoxidharz **) | 100 | 61,75 | 0,25 | 0,25/1 | 62 | 57 |
| Festepoxidharz ***) | 100 | 23,7 | 0,25 | 0,25/1 | 69,6 | 77. |

*) Führt man die Härtung in jeweils zweistündigen Zyklen bei 120 °C, 150 °C und 180 °C durch, so erhält man höhere $T_g$-Werte des Produktes (65,5 °C bzw. 83 °C)
**) Epoxidharz auf Basis von Bisphenol A ; Epoxid-Aequivalent = 5,25 (Val/kg) ; $\bar{M}_n$ = 350
***) Epoxidharz auf Basis von Bisphenol A ; Epoxid-Aequivalent : 2,0 (Val/kg), $\bar{M}_n$ ~ 1000.

Tabelle 2

| Typ Epoxidharz | Formulierung (Gew.Teile) | | | Adhäsion (N/mm²) | $\eta^{40}$ (mPas) |
|---|---|---|---|---|---|
| | Epoxidharz | Härter | Härtungsbeschleuniger | | |
| Flüssigepoxidharz *) | 20 | 8,03 | 0,05 | 54,8 | 3410 |
| Flüssigepoxidharz *) | 20 | 10,71 | 0,05 | 65,2 | 4051 |
| Flüssig epoxidharz *) | 20 | 13,39 | 0,05 | 68,0 | 4660 |
| Flüssigepoxidharz *) | 20 | 16,07 | 0,05 | 64,0 | 4700 |

*) Epoxidharz auf Basis von Bisphenol A ; Epoxid-Aequivalent : 5,25 (Val/kg)

B. Messung der Adhäsion von Klebstofformulierungen in Abhängigkeit der Lagerzeit

Eine Klebstofformulierung bestehend aus 100 Gew. Teilen Flüssigepoxidharz (auf Bisphenol A Basis mit einem Epoxid-Aequivalent von 5,33 Val/kg), aus 61,34 Gew.-Teilen 2,2'-Bis(3-acetylthiopropyl-4-hydroxyphenyl)-propan als Härter und aus 0,25 Teilen 2-Phenylimidazol als Härtungsbeschleuniger wird hergestellt, indem man das Harz und den Härter bei 85 °C mischt, auf 50 °C abkühlt und dann den Härtungsbeschleuniger zusetzt. Die Formulierung wird bei 6 °C bzw. bei 20 °C gelagert.

Zu den unterschiedlichen Zeiten werden das Epoxid-Aequivalent der Mischung (vgl. Kunststoffe 51, 714, 1961) und die Adhäsion der gehärteten Mischung (vgl. Anwendungsbeispiel A) gemessen.

Die Klebstellen werden vor dem Torsionsversuch jeweils zwei Stunden bei 120 °C gehärtet. Die Ergebnisse sind in Tabelle 3 dargestellt.

Tabelle 3

| Lagerzeit (Tage) bzw. (Monate) | Lagertemperatur | | | |
|---|---|---|---|---|
| | 6°C | | 20°C | |
| | Epoxid-Aequivalent (Val/kg) | Adhäsion ($N/mm^2$) | Epoxid-Aequivalent (Val/kg) | Adhäsion ($N/mm^2$) |
| 0 | 3,33 | 66 ± 2,2 | 3,33 | 66 ± 2,2 |
| 8 | 3,16 | - | 2,81 | - |
| 57 | 2,74 | 62 ± 3 | 1,31 | 52 ± 6 |
| 7,5 Monate | - | - | 0,48 | 39 ± 10 |

C. Kochwasserbeständigkeit von Al/Al-Verklebungen

Es werden Klebstofformulierungen bestehend aus Epoxidharz, Härter und Härtungsbeschleuniger hergestellt (Mengen vergl. untenstehende Tabelle).

Dazu werden das Flüssigepoxidharz und der Härter [2,2'-Bis-(3-acetylthiopropyl-4-hydroxyphenyl)-propan gemäss Beispiel 1] bei 85 °C gelöst, auf 50 °C abgekühlt und der Härtungsbeschleuniger (2-Phenylimidazol) zugesetzt.

Im Falle des Festepoxidharzes wird der Härter bei 100 °C zugegeben und der Härtungsbeschleuniger bei 70 °C.

Mit diesen Formulierungen werden Al/Al-Verklebungen hergestellt. Die Härtung erfolgt zwei Stunden bei 120 °C und dann 30 Minuten bei 180 °C.

Als Qualitätskriterium der Klebverbindungen wird die Zugscherfestigkeit nach DIN 53283 (auf Anticorodal B) gemessen.

Die Proben werden unmittelbar nach der Härtung bzw. nach definierten Zeitintervallen der Kalt- oder Heisswasserlagerung geprüft. Die Ergebnisse sind in Tabelle 4 dargestellt.

(Siehe Tabelle 4 Seite 10 f.)

Tabelle 4

| Typ Epoxidharz | Formulierung (Gew.Teil) | | | Zugscherfestigkeit ($N/mm^2$) nach Lagerung in Tagen (T) | | | | | | | |
|---|---|---|---|---|---|---|---|---|---|---|---|
| | Harz | Härter | Härtungsbeschl. | Kaltwasserlagerung (23°C) | | | | Heisswasserlagerung (90°C) | | | |
| | | | | 0.T. | 30.T. | 60.T. | 90.T. | 0.T. | 30.T. | 60.T. | 90.T. |
| Flüssigepoxidharz *) | 100 | 62 | 0,25 | 20-22 | 15-17 | 15-17 | 13-16 | 20-22 | 18-20 | 16-19 | 16-18 |
| Festepoxidharz **) | 100 | 25 | 0,25 | 21-23 | 18-20 | 18-21 | 17-20 | 21-23 | 21-23 | 19-21 | 18-21 |

*) Epoxidharz auf Basis von Bisphenol A ; Epoxid-Aequivalent = 5,25 (Val/kg) ; $\bar{M}_n$ = 380
**) Epoxidharz auf Basis von Bisphenol A ; Epoxid-Aequivalent = 2,0 (Val/kg) ; $\bar{M}_n$ ~ 1 000

**Patentansprüche** (für die Vertragsstaaten : DE, GB, FR, CH, LI, IT, NL)

1. Verbindungen der Formel I und II

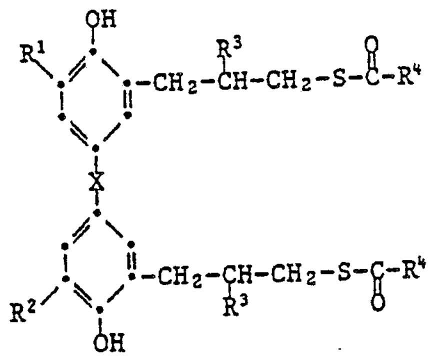

(I)

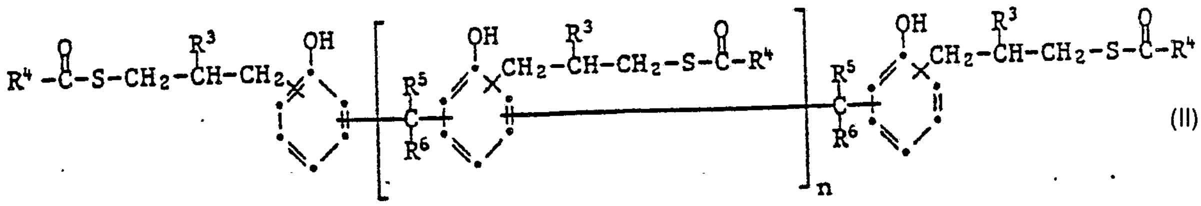

(II)

worin $R^1$ und $R^2$ unabhängig voneinander Wasserstoff, $C_1$-$C_{18}$Alkyl, Cyclohexyl, Phenyl, Benzyl oder Tolyl bedeuten oder ein Rest $—CH_2—CHR^3—CH_2—S—CO—R^4$ sind, $R^3$ Wasserstoff oder Methyl ist, $R^4$ $C_1$-$C_{18}$Alkyl, Cyclohexyl, Phenyl, Benzyl oder Tolyl ist, X $—CR^5R^6—$, —S—, —SO—, $—SO_2—$ oder $—(CH_3)C[—(CH_2)_m—COOR^7]—$ bedeutet, $R^5$ und $R^6$ unabhängig voneinander Wasserstoff oder $C_1$-$C_6$Alkyl sind, $R^7$ $C_1$-$C_{18}$-Alkyl ist, m 1 oder 2 bedeutet und n eine ganze Zahl von 1 bis 10 ist.

2. Verbindungen der Formel I oder II gemäss Anspruch 1, worin $R^3$ Wasserstoff bedeutet.

3. Verbindungen der Formel I gemäss Anspruch 1, worin $R^1$ und $R^2$ unabhängig voneinander Wasserstoff oder $C_1$-$C_{12}$Alkyl sind, $R^3$ Wasserstoff bedeutet, $R^4$ $C_1$-$C_4$Alkyl ist, X eine Gruppe $—CR^5R^6—$ darstellt und worin $R^5$ und $R^6$ unabhängig voneinander Wasserstoff oder Methyl sind.

4. Verbindungen der Formel I gemäss Anspruch 1, worin $R^1$, $R^2$ und $R^3$ Wasserstoff bedeuten, $R^4$ Methyl ist und X eine Gruppe $—CH_2—$, $—CH(CH_3)—$ oder $—C(CH_3)_2—$ darstellt.

5. Verbindungen der Formel I gemäss Anspruch 1, worin $R^1$ und $R^2$ eine Gruppe $—CH_2—CH(R^3)—CH_2—S—CO—R^4$ darstellen.

6. Verbindungen der Formel II gemäss Anspruch 1, worin $R^3$ Wasserstoff ist, $R^4$ Methyl bedeutet, $—CR^5R^6—$ eine Gruppe $—CH_2—$, $—CH(CH_3)—$ oder $—C(CH_3)_2—$ darstellt und worin n eine ganze Zahl von 1 bis 4 ist.

7. Verbindungen der Formel II gemäss Anspruch 1, worin die Gruppen $—CH_2—CHR^3—CH_2—S—CO—R^4$ sich jeweils in ortho-Position zur phenolischen Hydroxylgruppe befinden.

8. Zusammensetzungen enthaltend

a) ein Epoxidharz mit durchschnittlich mehr als einer Epoxidgruppe im Molekül oder ein noch schmelzbares und/oder lösliches härtbares Vorkondensat des Epoxidharzes (sogenannte « B-Stufe »), und

b) mindestens eine Verbindung der Formel I und/oder II gemäss Anspruch 1.

9. Verfahren zur Herstellung von Verbindungen der Formel I oder II gemäss Anspruch 1, dadurch gekennzeichnet, dass man Verbindungen der Formel III oder IV

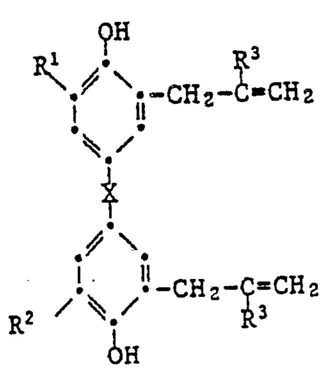

(III)

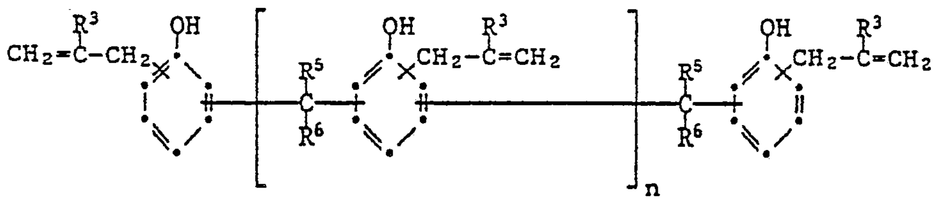

(IV)

mit einem dem Gehalt an allylständigen Doppelbindungen im wesentlichen entsprechenden molaren Anteil an Thiocarbonsäure der Formel V

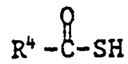

(V)

umsetzt, wobei die Reste $R^1$ $R^2$ $R^3$, X, $R^4$, $R^5$ und $R^6$, sowie der Index n die in Anspruch 1 definierte Bedeutung besitzen, wobei $R^1$ und $R^2$ allerdings anstelle von $—CH_2—CHR^3—CH_2—S—CO—R^4$ in diesem Falle $—CH_2—CR^3=CH_2$ sind.

10. Verwendung von Mischungen enthaltend

a) ein Epoxidharz mit durchschnittlich mehr als einer Epoxidgruppe im Molekül, und

b) mindestens eine Verbindung der Formel I und/oder II gemäss Anspruch 1

als Einkomponentenklebstofformulierung.

11. Gehärtete Produkte erhältlich durch Erwärmen von Zusammensetzungen gemäss Anspruch 8.

**Patentansprüche** (für den Vertragstaat AT)

1. Zusammensetzungen enthaltend

a) ein Epoxidharz mit durchschnittlich mehr als einer Epoxidgruppe im Molekül oder ein noch schmelzbares und/oder lösliches härtbares Vorkondensat des Epoxidharzes (sogenannte « B-Stufe »), und

b) mindestens eine Verbindung der Formel I und/oder II

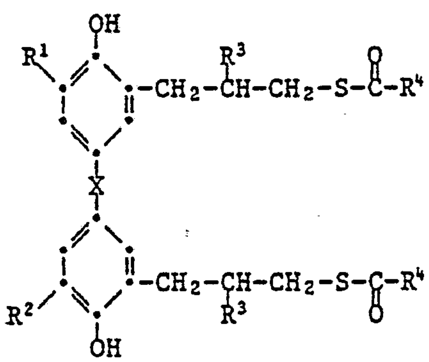

(I)

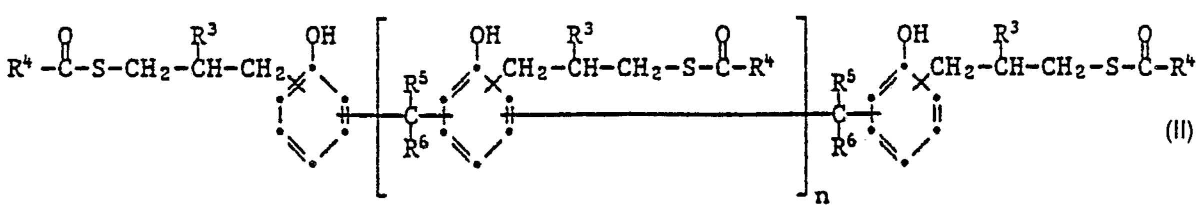

(II)

worin $R^1$ und $R^2$ unabhängig voneinander Wasserstoff, $C_1$-$C_{18}$Alkyl, Cyclohexyl, Phenyl, Benzyl oder Tolyl bedeuten oder ein Rest $—CH_2—CHR^3—CH_2—S—CO—R^4$ sind, $R^3$ Wasserstoff oder Methyl ist, $R^4$ $C_1$-$C_{18}$Alkyl, Cyclohexyl, Phenyl, Benzyl oder Tolyl ist, X $—CR^5R^6—$, $—S—$, $—SO—$, $—SO_2—$ oder $—(CH_3)C[—(CH_2)_m—COOR^7]—$ bedeutet, $R^5$ und $R^6$ unabhängig voneinander Wasserstoff oder $C_1$-$C_6$Alkyl sind, $R^7$ $C_1$-$C_{18}$-Alkyl ist, m 1 oder 2 bedeutet und n eine ganze Zahl von 1 bis 10 ist.

2. Verfahren zur Herstellung von Verbindungen der Formel I oder II gemäss Anspruch 1, dadurch gekennzeichnet, dass man Verbindungen der Formel III oder IV

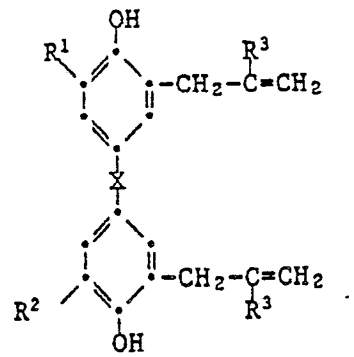

(III)

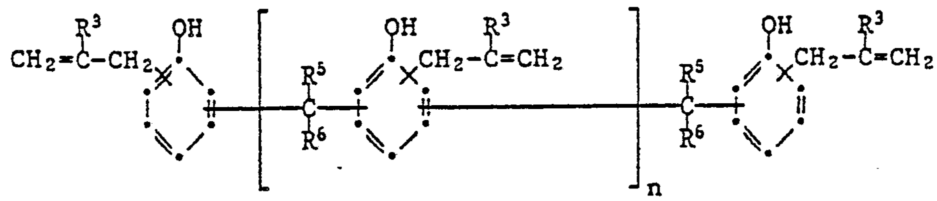

(IV)

mit einem dem Gehalt an allylständigen Doppelbindungen im wesentlichen entsprechenden molaren Anteil an Thiocarbonsäure der Formel V

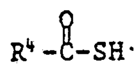

(V)

umsetzt, wobei die Reste $R^1$ $R^2$ $R^3$, X, $R^4$, $R^5$ und $R^6$, sowie der Index n die in Anspruch 1 definierte Bedeutung besitzen, wobei $R^1$ und $R^2$ allerdings anstelle von $-CH_2-CHR^3-CH_2-S-CO-R^4$ in diesem Falle $-CH_2-CR^3=CH_2$ sind.

3. Verfahren gemäss Anspruch 2, worin $R^3$ Wasserstoff bedeutet.

4. Verfahren zur Herstellung von Verbindungen der Formel I gemäss Anspruch 2, worin $R^1$ und $R^2$ unabhängig voneinander Wasserstoff oder $C_1$-$C_{12}$Alkyl sind, $R^3$ Wasserstoff bedeutet, $R^4$ $C_1$-$C_4$Alkyl ist, X eine Gruppe $-CR^5R^6-$ darstellt und worin $R^5$ und $R^6$ unabhängig voneinander Wasserstoff oder Methyl sind.

5. Verfahren zur Herstellung von Verbindungen der Formel I gemäss Anspruch 2, worin $R^1$, $R^2$ und $R^3$ Wasserstoff bedeuten, $R^4$ Methyl ist und X eine Gruppe $-CH_2-$, $-CH(CH_3)-$ oder $-C(CH_3)_2-$ darstellt.

6. Verfahren zur Herstellung von Verbindungen der Formel I gemäss Anspruch 2, worin $R^1$ und $R^2$ eine Gruppe $-CH_2-CH(R^3)-CH_2-S-CO-R^4$ darstellen.

7. Verfahren zur Herstellung von Verbindungen der Formel II gemäss Anspruch 2, worin $R^3$ Wasserstoff ist, $R^4$ Methyl bedeutet, $-CR^5R^6-$ eine Gruppe $-CH_2-$, $-CH(CH_3)-$ oder $-C(CH_3)_2-$ darstellt und worin n eine ganze Zahl von 1 bis 4 ist.

8. Verfahren zur Herstellung von Verbindungen der Formel II gemäss Anspruch 2, worin die Gruppen $-CH_2-CHR_3-CH_2-S-CO-R^4$ sich jeweils in ortho-Position zur phenolischen Hydroxylgruppe befinden.

9. Verwendung von Mischungen enthaltend

a) ein Epoxidharz mit durchschnittlich mehr als einer Epoxidgruppe im Molekül, und

b) mindestens eine Verbindung der Formel I und/oder II gemäss Anspruch 1

als Einkomponentenklebstofformulierung.

**Claims** (for the Contracting States : DE, GB, FR, CH, LI, IT, NL)

1. A compound of formula I or II

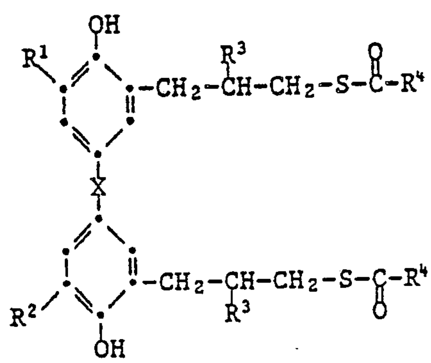

(I)

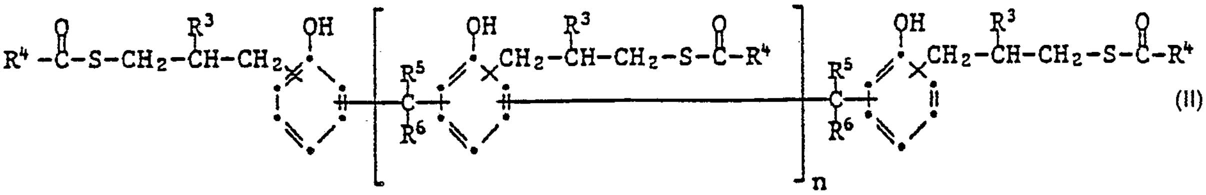

(II)

wherein $R^1$ and $R^2$ are each independently of the other hydrogen, $C_1$-$C_{18}$alkyl, cyclohexyl, phenyl, benzyl or tolyl, or are a $—CH_2—CHR^3—CH_2—S—CO—R^4$ radical, $R^3$ is hydrogen or methyl, $R^4$ is $C_1$-$C_{18}$alkyl, cyclohexyl, phenyl, benzyl or tolyl, X is $—CR^5R^6—$, $—S—$, $—SO—$, $—SO_2—$ or $—(CH_3)C[—(CH_2)_m—COOR^7]—$, in which $R^5$ and $R^6$ are each independently of the other hydrogen or $C_1$-$C_6$alkyl, $R^7$ is $C_1$-$C_{18}$alkyl, m is 1 or 2 and n is an integer from 1 to 10.

2. A compound of formula I or II according to claim 1, wherein $R^3$ is hydrogen.

3. A compound of formula I according to claim 1, wherein $R^1$ and $R^2$ are each independently of the other hydrogen or $C_1$-$C_{12}$alkyl, $R^3$ is hydrogen, $R^4$ is $C_1$-$C_4$alkyl, X is a $—CR^5R^6$ group, and wherein $R^5$ and $R^6$ are each independently of the other hydrogen or methyl.

4. A compound of formula I according to claim 1, wherein $R^1$, $R^2$ and $R^3$ are hydrogen, $R^4$ is methyl, and X is a $—CH_2—$, $—CH(CH_3)—$ or $—C(CH_3)_2$ group.

5. A compound of formula I according to claim 1, wherein $R^1$ and $R^2$ are a $—CH_2—CH(R^3)—CH_2—S—CO—R^4$ group.

6. A compound of formula II according to claim 1, wherein $R^3$ is hydrogen, $R^4$ is methyl, $—CR^5R^6—$ is a $—CH_2—$, $—CH(CH_3)—$ or $—C(CH_3)_2—$ group and n is an integer from 1 to 4.

7. A compound of formula II according to claim 1, wherein the $—CH_2—CHR^3—CH_2—S—CO—R^4$ groups are each ortho-positioned with respect to the phenolic hydroxyl group.

8. A composition containing

a) an epoxy resin having on average more than one epoxy group in the molecule or a still fusible and/or soluble curable precondensate of said epoxy resin (so-called B-stage), and

b) at least one compound of formula I and/or II according to claim 1.

9. A process for the preparation of a compound of formula I or II according to claim 1, which comprises reacting a compound of formula III or IV

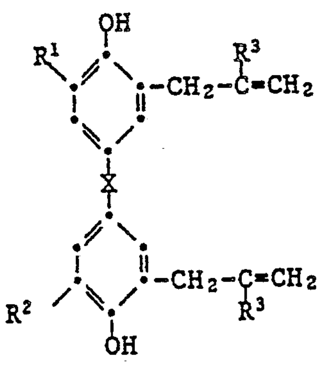

(III)

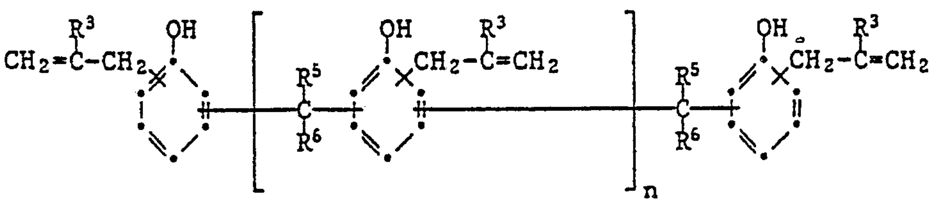

(IV)

with a molar amount of a thiocarboxylic acid of formula V

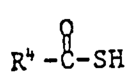

(V)

which is substantially proportional to the content of allylic double bonds, where the radicals $R^1$, $R^2$, $R^3$, X, $R^4$, $R^5$ and $R^6$, as well as the index n are as defined in claim 1 except that in this case $R^1$ and $R^2$ are $—CH_2—CR^3{=}CH_2$ instead of $—CH_2—CHR^3—CH_2—S—CO—R^4$.

10. The use of a mixture containing

a) an epoxy resin having on average more than one epoxy group in the molecule and

b) at least one compound of formula I and/or II according to claim 1

as a single component adhesive formulation.

11. A cured product obtainable by heating a composition according to claim 8.

**Claims** (for the Contracting State AT)

1. A composition containing

a) an epoxy resin having on average more than one epoxy group in the molecule or a still fusible and/or soluble curable precondensate of said epoxy resin (so-called B-stage), and

b) at least one compound of formula I and/or II

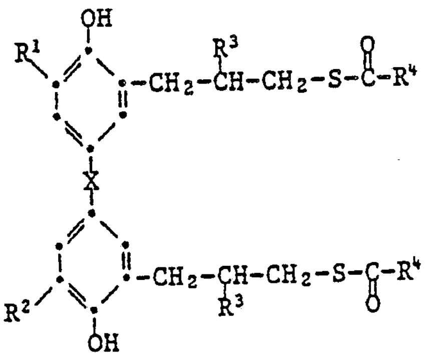

(I)

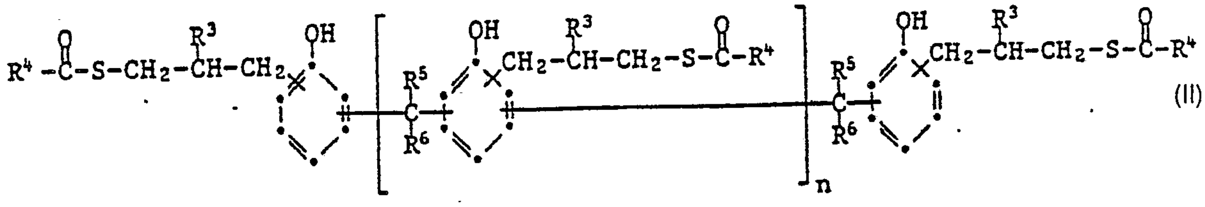

(II)

wherein $R^1$ and $R^2$ are each independently of the other hydrogen, $C_1$-$C_{18}$alkyl, cyclohexyl, phenyl, benzyl or tolyl, or are a $—CH_2—CHR^3—CH_2—S—CO—R^4$ radical, $R^3$ is hydrogen or methyl, $R^4$ is $C_1$-$C_{18}$alkyl, cyclohexyl, phenyl, benzyl or tolyl, X is $—CR^5R^6—$, $—S—$, $—SO—$, $—SO_2—$ or $—(CH_3)C[—(CH_2)_m—COOR^7]—$, in which $R^5$ and $R^6$ are each independently of the other hydrogen or $C_1$-$C_6$alkyl, $R^7$ is $C_1$-$C_{18}$alkyl, m is 1 or 2 and n is an integer from 1 to 10.

2. A process for the preparation of a compound of formula I or II according to claim 1, which comprises reacting a compound of formula III or IV

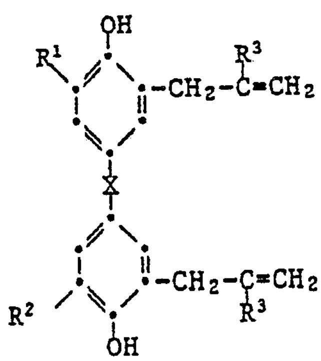

(III)

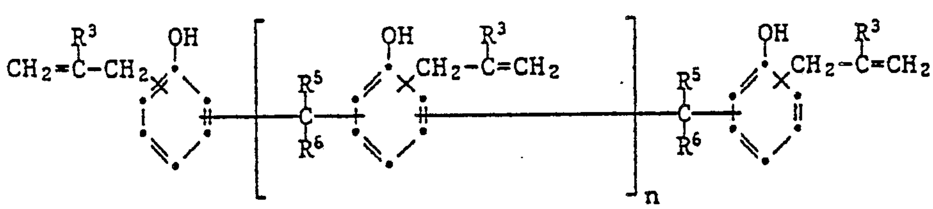

(IV)

with a molar amount of a thiocarboxylic acid of formula V

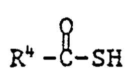

(V)

which is substantially proportional to the content of allylic double bonds, where the radicals $R^1$, $R^2$, $R^3$, X, $R^4$, $R^5$ and $R^6$, as well as the index n are as defined in claim 1 except that in this case $R^1$ and $R^2$ are $—CH_2—CR^3=CH_2$ instead of $—CH_2—CHR^3—CH_2—S—CO—R^4$.

3. A process according to claim 2, wherein $R^3$ is hydrogen.

4. A process for the preparation of a compound of formula I according to claim 2, wherein $R^1$ and $R^2$ are each independently of the other hydrogen or $C_1$-$C_{12}$alkyl, $R^3$ is hydrogen, $R^4$ is $C_1$-$C_4$alkyl, X is a $—CR^5R^6$ group, and wherein $R^5$ and $R^6$ are each independently of the other hydrogen or methyl.

5. A process for the preparation of a compound of formula I according to claim 2, wherein $R^1$, $R^2$ and $R^3$ are hydrogen, $R^4$ is methyl, and X is a $—CH_2—$, $—CH(CH_3)—$ or $—C(CH_3)_2$ group.

6. A process for the preparation of a compound of formula I according to claim 2, wherein $R^1$ and $R^2$ are a $—CH_2—CH(R^3)—CH_2—S—CO—R^4$ group.

7. A process for the preparation of a compound of formula II according to claim 2, wherein $R^3$ is hydrogen, $R^4$ is methyl, $—CR^5R^6—$ is a $—CH_2—$, $—CH(CH_3)—$ or $—C(CH_3)_2—$ group and n is an integer from 1 to 4.

8. A process for the preparation of a compound of formula II according to claim 2, wherein the $—CH_2—CHR^3—CH_2—S—CO—R^4$ groups are each ortho-positioned with respect to the phenolic hydroxyl group.

9. The use of a mixture containing

a) an epoxy resin having on average more than one epoxy group in the molecule and

b) at least one compound of formula I and/or II according to claim 1

as a single component adhesive formulation.

**Revendications** (pour les Etats contractants : DE, GB, FR, CH, LI, IT, NL)

1. Composés répondant à l'une des formules I et II :

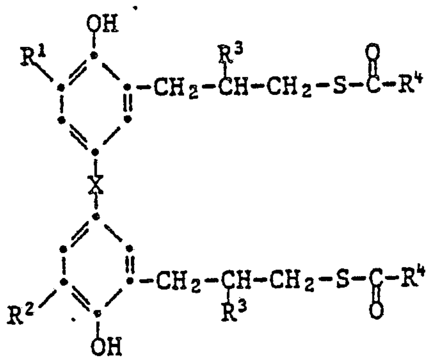

(I)

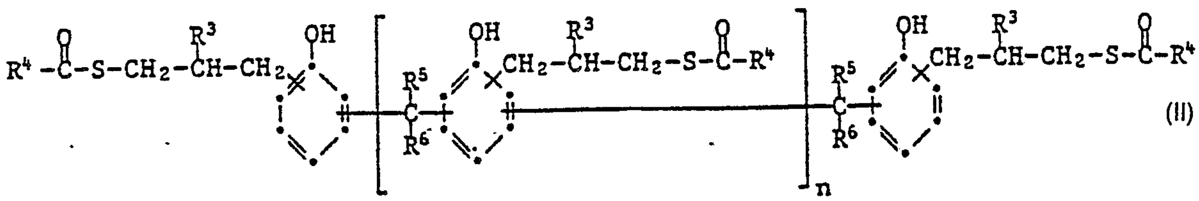

(II)

dans lesquelles $R^1$ et $R^2$ représentent chacun, indépendamment l'un de l'autre, l'hydrogène, un alkyle en $C_1$-$C_{18}$, un cyclohexyle, un phényle, un benzyle, un tolyle, ou un radical $—CH_2—CHR^3—CH_2—S—CO—R^4$, $R^3$ représente l'hydrogène ou un méthyle, $R^4$ représente un alkyle en $C_1$-$C_{18}$, un cyclohexyle, un phényle, un benzyle ou un tolyle, X représente un radical $—CR^5R^6—$, $—S—$, $—SO—$, $—SO_2—$ ou $—(CH_3)C[—(CH_2)_m—COOR^7]—$, $R^5$ et $R^6$ représentent chacun, indépendamment l'un de l'autre, l'hydrogène ou un alkyle en $C_1$-$C_6$, $R^7$ représente un alkyle en $C_1$-$C_{18}$, m est égal à 1 ou à 2 et n représente un nombre entier de 1 à 10.

2. Composés de formule I ou II selon la revendication 1 dans lesquels $R^3$ représente l'hydrogène.

3. Composés de formule I selon la revendication 1 dans lesquels $R^1$ et $R^2$ représentent chacun, indépendamment l'un de l'autre, l'hydrogène ou un alkyle en $C_1$-$C_{12}$, $R^3$ représente l'hydrogène, $R^4$ un alkyle en $C_1$-$C_4$, X un radical $—CR^5R^6—$, et $R^5$ et $R^6$ représentent chacun, indépendamment l'un de l'autre, l'hydrogène ou un méthyle.

4. Composés de formule I selon la revendication 1 dans lesquels $R^1$, $R^2$ et $R^3$ représentent chacun l'hydrogène, $R^4$ représente un méthyle et X un radical $—CH_2—$, $—CH(CH_3)—$ ou $—C(CH_3)_2—$.

5. Composés de formule I selon la revendication 1 dans lesquels $R^1$ et $R^2$ représentent chacun un radical $—CH_2—CH(R^3)—CH_2—S—CO—R^4$.

6. Composés de formule II selon la revendication 1 dans lesquels $R^3$ représente l'hydrogène, $R^4$ représente un méthyle, le radical $—CR^5R^6—$ représente un radical $—CH_2—$, $—CH(CH_3)—$ ou $—C(CH_3)_2—$, et n désigne un nombre entier de 1 à 4.

7. Composés de formule II selon la revendication 1 dans lesquels les radicaux $—CH_2—CHR^3—CH_2—S—CO—R^4$ se trouvent chacun en position ortho par rapport au radical hydroxy phénolique.

8. Compositions qui contiennent :

a) une résine époxydique renfermant en moyenne plus d'un radical époxy par molécule, ou un précondensat durcissable, encore fusible et/ou soluble, de la résine époxydique (« stade B »), et

b) au moins un composé répondant à l'une des formules I et II selon la revendication 1.

9. Procédé de préparation de composés de formule I ou II selon la revendication 1, procédé caractérisé en ce qu'on fait réagir des composés de formule III ou IV :

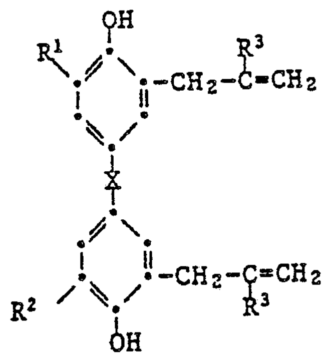

(III)

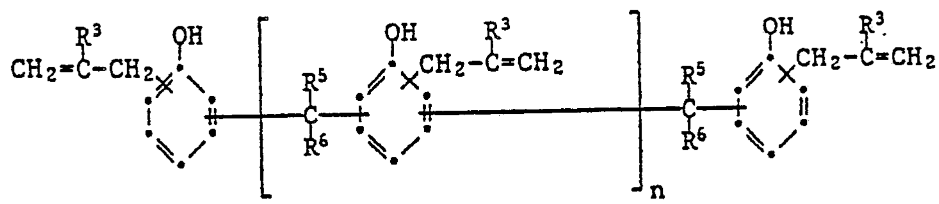

(IV)

avec une quantité molaire d'un acide carbothioïque de formule V :

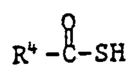

(V)

qui correspond essentiellement à la quantité des doubles liaisons allyliques, les symboles $R^1$, $R^2$, $R^3$, X, $R^4$, $R^5$ et $R^6$ et l'indice n ayant les significations données à la revendication 1 à cette différence près toutefois que, dans la définition de $R^1$ et $R^2$, il faut remplacer le radical $—CH_2—CHR^3—CH_2—S—CO—R^4$ par le radical $—CH_2—CR^3{=}CH_2$.

10. Application de mélanges contenant :

a) une résine époxydique qui renferme en moyenne plus d'un radical époxy par molécule, et

b) au moins un composé répondant à l'une des formules I et II selon la revendication 1,

comme colles à une seule composante.

11. Produits durcis qui ont été obtenus par chauffage de compositions selon la revendication 8.

**Revendications** (pour l'Etat contractant AT)

1. Compositions qui contiennent :

a) une résine époxydique renfermant en moyenne plus d'un radical époxy par molécule, ou un précondensat durcissable, encore fusible et/ou soluble, de la résine époxydique (« stade B »), et

b) au moins un composé répondant à l'une des formules I et II :

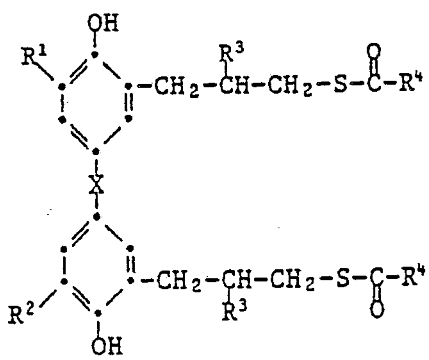

(I)

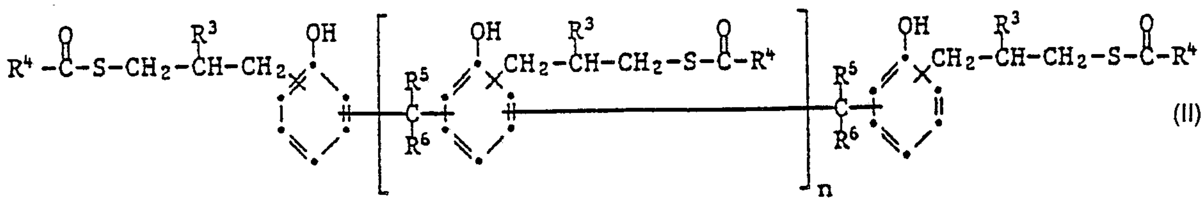

(II)

dans lesquelles $R^1$ et $R^2$ représentent chacun, indépendamment l'un de l'autre, l'hydrogène, un alkyle en $C_1$-$C_{18}$, un cyclohexyle, un phényle, un benzyle, un tolyle, ou radical $—CH_2—CHR^3—CH_2—S—CO—R^4$, $R^3$ représente l'hydrogène ou un méthyle, $R^4$ représente un alkyle en $C_1$-$C_{18}$, un cyclohexyle, un phényle, un benzyle ou un tolyle, X représente un radical $—CR^5R^6—$, —S—, —SO—, $—SO_2—$ ou $—(CH_3)C[—(CH_2)_m—COOR^7]—$, $R^5$ et $R^6$ représentent chacun, indépendamment l'un de l'autre, l'hydrogène ou un alkyle en $C_1$-$C_6$, $R^7$ représente un alkyle en $C_1$-$C_{18}$, m est égal à 1 ou à 2 et n représente un nombre entier de 1 à 10.

2. Procédé de préparation de composés de formule I ou II selon la revendication 1, procédé caractérisé en ce qu'on fait réagir des composés de formule III ou IV :

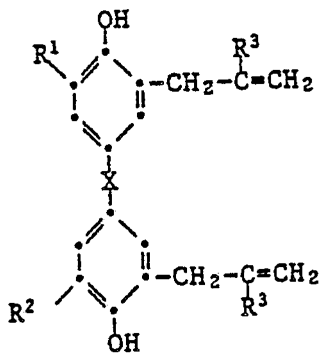

(III)

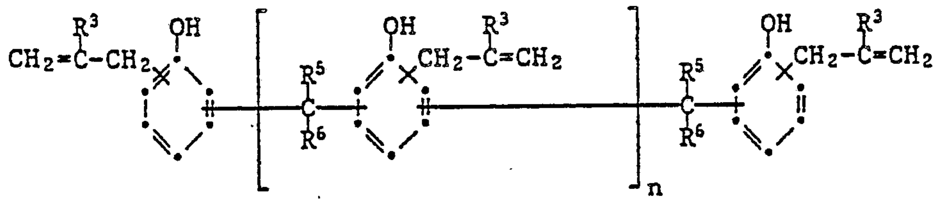

(IV)

avec une quantité d'un acide carbothioïque de formule V :

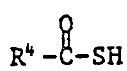

(V)

qui correspond essentiellement, en moles, à la quantité des doubles liaisons allyliques, les symboles $R^1$, $R^2$, $R^3$, X, $R^4$, $R^5$ et $R^6$ ainsi que l'indice n ayant les significations indiquées à la revendication 1 à cette différence près toutefois que, dans la définition de $R^1$ et de $R^2$, il faut remplacer le radical $—CH_2—CHR^3—CH_2—S—CO—R^4$ par le radical $—CH_2—CR^3{=}CH_2$.

3. Procédé selon la revendication 2 dans lequel $R^3$ représente l'hydrogène.

4. Procédé selon la revendication 2 pour la préparation de composés de formule I dans lesquels $R^1$ et $R^2$ représentent chacun, indépendamment l'un de l'autre, l'hydrogène ou un alkyle en $C_1$-$C_{12}$, $R^3$ représente l'hydrogène, $R^4$ un alkyle en $C_1$-$C_4$, X un radical $—CR^5R^6—$, et $R^5$ et $R^6$ représentent chacun, indépendamment l'un de l'autre, l'hydrogène ou un méthyle.

5. Procédé selon la revendication 2 pour la préparation de composés de formule I dans lesquels $R^1$, $R^2$ et $R^3$ représentent chacun l'hydrogène, $R^4$ représente un méthyle et X un radical $—CH_2—$, $—CH(CH_3)—$ ou $—C(CH_3)_2—$.

6. Procédé selon la revendication 2 pour la préparation de composés de formule I dans lesquels $R^1$ et $R^2$ représentent chacun un radical $—CH_2—CH(R^3)—CH_2—S—CO—R^4$.

7. Procédé selon la revendication 2 pour la préparation de composés de formule II dans lesquels $R^3$ représente l'hydrogène, $R^4$ représente un méthyle, le radical $—CR^5R^6—$ représente un radical $—CH_2—$, $—CH(CH_3)—$ ou $—C(CH_3)_2—$, et n désigne un nombre entier de 1 à 4.

8. Procédé selon la revendication 2 pour la préparation de composés de formule II dans lesquels les

radicaux $-CH_2-CHR^3-CH_2-S-CO-R^4$ se trouvent chacun en position ortho par rapport au radical hydroxy phénolique.

9. Application de mélanges qui contiennent :

a) une résine époxydique qui renferme en moyenne plus d'un radical époxy par molécule, et

b) au moins un composé répondant à l'une des formules I et II selon la revendication 1,

comme colles à une seule composante.